# EUROPEAN PATENT APPLICATION

(11) **EP 4 450 520 A1**
(43) Date of publication of application: **23.10.2024**
(21) Application number: 22906592.5
(22) Date of filing: 14.12.2022
(51) Int. Cl.: C07K 16/28, A61K 38/17, A61P 35/00, C07K 19/00, A61K 39/395, C12N 15/13, C12P 21/06

(54) **PD-L1 ANTIGEN BINDING PROTEIN AND APPLICATION THEREOF**

(30) Priority: 15.12.2021 CN 202111530863
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: WEI, Xiaoyue, Shanghai 201203 (CN); ZHU, Xiangyang, Shanghai 201203 (CN); YU, Haijia, Shanghai 201203 (CN); CHEN, Shi, Shanghai 201203 (CN); WANG, Yanting, Shanghai 201203 (CN); REN, Xiaochen, Shanghai 201203 (CN); CUI, Xiaopei, Shanghai 201203 (CN); JIA, Huifeng, Shanghai 201203 (CN)
(74) Representative: Weickmann & Weickmann PartmbB
(86) International application number: PCT/CN2022/138898
(87) International publication number: WO 2023/109847

(57) **Abstract**

A PD-L1 antigen binding protein and an application thereof. The antigen binding protein has one or more of the following properties: binding to a PD-L1 protein at an EC₅₀ value of 0.07 µg/ml or above; blocking binding between the PD-L1 protein and a PD-1 protein; promoting the activation of a T cell; and inhibiting the volume increase of the in vivo tumor. An application of the antigen binding protein in the preparation of a drug for treating tumor.

## Description

### Field of the Invention

The present application relates to the field of biomedicine, and particularly to a PD-L1 antigen binding protein and a use thereof.

### Background of the Invention

Programmed cell death protein-1 (PD-1), a negative costimulatory molecule, is a superfamily of CD28 immunoglobulin. PD-1 possesses two natural ligands, programmed death ligand-1 (PD-L1) and PD-L2. PD-L1 is an important negative immunomodulatory factor, also known as B7-H1, the binding of which to PD-1 mediates the co-inhibitory signal of T cell activation, inhibits T cell activation and proliferation, and induces T cell apoptosis.

Immunomodulation targeting PD-1/PD-L1 is of great significance in combating tumors. PD-L1 is over-expressed on the surface of various tumor cells and binds to PD-1 molecules on T cells, inducing T cell apoptosis and thus aiding tumor immune evasion. In recent years, 10 monoclonal antibody drugs targeting PD-1 or PD-L1 have been successively launched on the market.

However, more and more clinical data show that, PD-1/PD-L1 inhibitors only play an effective role in "hot tumors" with sufficient infiltration of T cells, macrophages, etc., and their average clinical effective rate ranges between 20% and 30%, indicating that there is still significant room for improvement in the indications and treatment regimens of PD-L1 inhibitors.

### Summary of the Invention

The present application provides a PD-L1 antigen binding protein and a use thereof.

In one aspect, the present application provides an isolated antigen binding protein, which has one or more of the following properties:
(1) capable of binding to a PD-L1 protein at an EC₅₀ value of 0.07 µg/ml or above;
(2) capable of blocking the binding of the PD-L1 protein to a PD-1 protein;
(3) capable of promoting the activation of a T cell; and
(4) capable of inhibiting the volume increase of a tumor in the body.

In some embodiments, the isolated antigen binding protein comprises an antibody or an antigen binding fragment thereof.

In some embodiments, the antigen binding fragment comprises Fab, Fab', F(ab)2, an Fv fragment, F(ab')2, scFv, di-scFv, VHH and/or dAb.

In some embodiments, the antibody is selected from the group consisting of a monoclonal antibody, a chimeric antibody, a humanized antibody, and a full human antibody.

In some embodiments, the isolated antigen binding protein competes with a reference antibody for binding to the PD-L1 protein, wherein the reference antibody comprises a heavy chain variable region VH and a light chain variable region VL, the VH of the reference antibody comprises a HCDR1, a HCDR2 and a HCDR3, the VL of the reference antibody comprises a LCDR1, a LCDR2 and a LCDR3, and the HCDR1 of the reference antibody comprises an amino acid sequence as set forth in SEQ ID NO: 1, the HCDR2 of the reference antibody comprises an amino acid sequence as set forth in SEQ ID NO: 21, the HCDR3 of the reference antibody comprises an amino acid sequence as set forth in SEQ ID NO: 22, the LCDR1 of the reference antibody comprises an amino acid sequence as set forth in SEQ ID NO: 6, the LCDR2 of the reference antibody comprises an amino acid sequence as set forth in SEQ ID NO: 23, and the LCDR3 of the reference antibody comprises an amino acid sequence as set forth in SEQ ID NO: 8.

In some embodiments, the isolated antigen binding protein comprises a HCDR1, and the HCDR1 comprises an amino acid sequence as set forth in SEQ ID NO: 1.

In some embodiments, the isolated antigen binding protein comprises a HCDR2, and the HCDR2 comprises an amino acid sequence as set forth in SEQ ID NO: 21.

In some embodiments, the isolated antigen binding protein comprises a HCDR2, and the HCDR2 comprises an amino acid sequence as set forth in SEQ ID NO: 2 or 4.

In some embodiments, the isolated antigen binding protein comprises a HCDR3, and the HCDR3 comprises an amino acid sequence as set forth in SEQ ID NO: 22.

In some embodiments, the isolated antigen binding protein comprises a HCDR3, and the HCDR3 comprises an amino acid sequence as set forth in SEQ ID NO: 3 or 5.

In some embodiments, the isolated antigen binding protein comprises a HCDR1, a HCDR2 and a HCDR3, wherein the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 3; or
wherein the HCDR1 comprises an amino acid sequence as set forth in SEQ ID NO: 1, the HCDR2 comprises an amino acid sequence as set forth in SEQ ID NO: 4, and the HCDR3 comprises an amino acid sequence as set forth in SEQ ID NO: 3; or
wherein the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 5; or
wherein the HCDR1 comprises an amino acid sequence as set forth in SEQ ID NO: 1, the HCDR2 comprises an amino acid sequence as set forth in SEQ ID NO: 4, and the HCDR3 comprises an amino acid sequence as set forth in SEQ ID NO: 5.

In some embodiments, the isolated antigen binding protein comprises a heavy chain variable region VH, and the VH comprises an amino acid sequence as set forth in any one of SEQ ID NOs: 11-16.

In some embodiments, the isolated antigen binding protein comprises a heavy chain constant region, and the heavy chain constant region is derived from a human IgG constant region.

In some embodiments, the heavy chain constant region is derived from a human IgG1 constant region, and the human IgG1 constant region comprises an amino acid sequence as set forth in SEQ ID NO: 55.

In some embodiments, the isolated antigen binding protein comprises a LCDR1, and the LCDR1 comprises an amino acid sequence as set forth in SEQ ID NO: 6.

In some embodiments, the isolated antigen binding protein comprises a LCDR2, and the LCDR2 comprises an amino acid sequence as set forth in SEQ ID NO: 23.

In some embodiments, the isolated antigen binding protein comprises a LCDR2, and the LCDR2 comprises an amino acid sequence as set forth in SEQ ID NO: 7, 9 or 10.

In some embodiments, the isolated antigen binding protein comprises a LCDR3, and the LCDR3 comprises an amino acid sequence as set forth in SEQ ID NO: 8.

In some embodiments, the isolated antigen binding protein comprises a LCDR1, a LCDR2 and a LCDR3, wherein the LCDR1 comprises an amino acid sequence as set forth in SEQ ID NO: 6, the LCDR2 comprises an amino acid sequence as set forth in SEQ ID NO: 7, and the LCDR3 comprises an amino acid sequence as set forth in SEQ ID NO: 8; or
wherein the LCDR1 comprises an amino acid sequence as set forth in SEQ ID NO: 6, the LCDR2 comprises an amino acid sequence as set forth in SEQ ID NO: 9, and the LCDR3 comprises an amino acid sequence as set forth in SEQ ID NO: 8; or
wherein the LCDR1 comprises an amino acid sequence as set forth in SEQ ID NO: 6, the LCDR2 comprises an amino acid sequence as set forth in SEQ ID NO: 10, and the LCDR3 comprises an amino acid sequence as set forth in SEQ ID NO: 8.

In some embodiments, the isolated antigen binding protein comprises a light chain variable region VL, wherein the VL comprises an amino acid sequence as set forth in any one of SEQ ID NOs: 17-20.

In some embodiments, the isolated antigen binding protein comprises an antibody light chain constant region, and the antibody light chain constant region comprises an amino acid sequence as set forth in SEQ ID NO: 56.

In some embodiments, the isolated antigen binding protein comprises a heavy chain variable region VH and a light chain variable region VL, wherein,
the VH comprises an amino acid sequence as set forth in SEQ ID NO: 11, and the VL comprises an amino acid sequence as set forth in SEQ ID NO: 17, or
the VH comprises an amino acid sequence as set forth in SEQ ID NO: 12, and the VL comprises an amino acid sequence as set forth in SEQ ID NO: 18, or
the VH comprises an amino acid sequence as set forth in SEQ ID NO: 13, and the VL comprises an amino acid sequence as set forth in SEQ ID NO: 18, or
the VH comprises an amino acid sequence as set forth in SEQ ID NO: 14, and the VL comprises an amino acid sequence as set forth in SEQ ID NO: 18, or
the VH comprises an amino acid sequence as set forth in SEQ ID NO: 15, and the VL comprises an amino acid sequence as set forth in SEQ ID NO: 18, or
the VH comprises an amino acid sequence as set forth in SEQ ID NO: 16, and the VL comprises an amino acid sequence as set forth in SEQ ID NO: 18, or
the VH comprises an amino acid sequence as set forth in SEQ ID NO: 13, and the VL comprises an amino acid sequence as set forth in SEQ ID NO: 19, or
the VH comprises an amino acid sequence as set forth in SEQ ID NO: 14, and the VL comprises an amino acid sequence as set forth in SEQ ID NO: 19, or
the VH comprises an amino acid sequence as set forth in SEQ ID NO: 15, and the VL comprises an amino acid sequence as set forth in SEQ ID NO: 19, or
the VH comprises an amino acid sequence as set forth in SEQ ID NO: 16, and the VL comprises an amino acid sequence as set forth in SEQ ID NO: 19, or
the VH comprises an amino acid sequence as set forth in SEQ ID NO: 14, and the VL comprises an amino acid sequence as set forth in SEQ ID NO: 20, or
the VH comprises an amino acid sequence as set forth in SEQ ID NO: 15, and the VL comprises an amino acid sequence as set forth in SEQ ID NO: 20, or
the VH comprises an amino acid sequence as set forth in SEQ ID NO: 16, and the VL comprises an amino acid sequence as set forth in SEQ ID NO: 20.

In another aspect, the present application provides a polypeptide that comprises the isolated antigen binding protein.

In another aspect, the present application provides an immunoconjugate that comprises the isolated antigen binding protein or the polypeptide.

In another aspect, the present application provides one or more isolated nucleic acid molecules that encode the isolated antigen binding protein.

In another aspect, the present application provides a vector that comprises the nucleic acid molecules.

In another aspect, the present application provides a cell that comprises the nucleic acid molecules or the vector.

In another aspect, the present application provides a method for preparing the isolated antigen binding protein, the method comprises culturing the cell under a condition enabling the expression of the isolated antigen binding protein.

In another aspect, the present application provides a pharmaceutical composition that comprises the isolated antigen binding protein, the nucleic acid molecules, the vector and/or the cell, and optionally a pharmaceutically acceptable carrier.

In another aspect, the present application provides a use of the isolated antigen binding protein, the nucleic acid molecules, the vector, the cell and/or the pharmaceutical composition in the preparation of a drug for preventing, alleviating, and/or treating a tumor.

In another aspect, the present application provides a method for preventing, alleviating, or treating a tumor, the method comprises administering to a subject in need thereof the isolated antigen binding protein, the nucleic acid molecules, the vector, the cell and/or the pharmaceutical composition.

In another aspect, the present application provides the isolated antigen binding protein that is used for preventing, alleviating, or treating a tumor.

In some embodiments, the tumor comprises a tumor with high expression of PD-1 or PD-L1.

In some embodiments, the tumor comprises a solid tumor.

In some embodiments, the tumor comprises colon cancer.

In another aspect, the present application provides a method for inhibiting the binding of a PD-1 protein to a PD-L1 protein, which comprises administering the isolated antigen binding protein and/or the polypeptide.

In another aspect, the present application provides a method for promoting the activation of a T cell, which comprises administering the isolated antigen binding protein and/or the polypeptide.

In another aspect, the present application provides a method for detecting the presence and/or content of a PD-L1 protein, which comprises administering the isolated antigen binding protein and/or the polypeptide.

In another aspect, the present application provides a kit that comprises the isolated antigen binding protein, the polypeptide, the immunoconjugate, the nucleic acid molecules, the vector, the cell and/or the pharmaceutical composition.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
FIG. 1 shows the blocking effect of a candidate chimeric antibody on the binding of free PD-1 to cell surface PD-L1.
FIG. 2 shows the blocking effect of a candidate chimeric antibody on the binding of free PD-1 to cell surface PD-1.
FIG. 3 shows the binding results of the antigen binding protein of the present application to cell surface PD-L1.
FIG. 4 shows the blocking of the antigen binding protein of the present application on the interaction of PD-1/PD-L1.
FIG. 5 shows the promotion of the antigen binding protein of the present application on the activation of T cells.
FIG. 6 shows the tumor volume of mouse animal models after being treated with the antigen binding protein of the present application.
FIG. 7 shows the body weight change of mouse animal models after being treated with the antigen binding protein of the present application.

### Detailed Description of the Embodiments

The implementation of the present application will be illustrated in the following specific examples, and other advantages and effects of the present application will be easily known by those skilled in the art from the content disclosed in the specification.

### Definition of Terms

In the present application, the term "PD-L1" generally refers to programmed death ligand 1 protein. PD-L1 is also referred to as cluster of differentiation 274 (CD274) or B7 homologue 1 (B7-H1), and is a protein encoded by the CD274 gene (in human). PD-L1 can bind its receptors, e.g., programmed death 1 (PD-1). The complexation of PD-L1 and PD-1 exerts immunosuppressive effects by inhibiting the proliferation of a T cell and producing cytokines IL-2 and IFN-γ. The term "PD-L1" covers any natural PD-L1 or modified PD-1 derived from any vertebrates, comprising mammals, such as primates (e.g., human and monkeys) and rodents (e.g., mice and rats). The term covers "full-length", unprocessed PD-L1 and any forms of PD-L1 produced from cell processing. PD-L1 may exist as transmembrane protein or soluble protein. The term also covers naturally occurring PD-L1 variants, e.g., splice variants or allelic variants. The basic structure of PD-L1 comprises 4 domains: an extracellular Ig-like V-domain and an Ig-like C2-domain, a transmembrane domain and a cytoplasmic domain. PD-L1 sequences are known in the art. For example, the information of human PD-L1 genes (comprising genomic DNA sequences) can be found under NCBI Gene ID No. 29126. The amino acid sequences of exemplary full-length human PD-L1 proteins can be found under NCBI accession No. NP_054862 or UniProt accession No. Q9NZQ7.

In the present application, the term "antigen binding protein" generally refers to a protein containing a binding antigen portion, and optionally, a scaffold or framework portion that allows the antigen-binding portion to adopt a conformation that facilitates the antigen-binding portion to bind to the antigen. The antigen binding protein may typically comprise an antibody light chain variable region (VL), an antibody heavy chain variable region (VH) or both the above, and functional fragments thereof. The variable region of a heavy chain and a light chain contains a binding domain interacting with the antigen. Examples of an antigen binding protein comprise, but not limited to, antibodies, antigen binding fragments, immunoconjugates, multispecific antibodies (e.g., bispecific antibodies), antibody fragments, antibody derivatives, antibody analogs or fusion proteins, etc., as long as they show the desired antigen binding activity.

In the present application, the term "antibody" generally refers to immunoglobulins reactive to specified proteins or peptides or fragments thereof. The antibody may be derived from antibodies of any type, comprising but not limited to IgG, IgA, IgM, IgD and IgE, and antibodies of any subtype (e.g., IgG1, IgG2, IgG3, and IgG4). The antibody may have a heavy chain constant region selected from, e.g., IgG1, IgG2, IgG3, or IgG4. The antibody may also have a light chain selected from, e.g., kappa (κ) or lambda (λ). The antibody of the present application may be derived from any species.

In the present application, the term "antigen binding fragment" generally refers to a portion of an antibody molecule containing an amino acid residue that interacts with an antigen and confers specificity and affinity to the antibody for the antigen. Examples of antigen binding fragments may comprise but not limited to, Fab, Fab', F(ab)₂, an Fv fragment, F(ab')₂, scFv, di-scFv and/or dAb. In the present application, the term "Fab" generally refers to fragments containing a heavy chain variable domain and a light chain variable domain, which may also contain a constant domain of the light chain and a first constant domain (CH1) of the heavy chain. The term "Fab'" generally refers to a fragment that differs from Fab by the addition of a small number of residues (comprising one or more cysteines from the hinge region of the antibody) to the carboxyl terminus of the heavy chain CH1 domain. The term "F(ab')₂" generally refers to a dimer of Fab', which is an antibody fragment containing two Fab fragments linked by a disulfide bridge in the hinge region. The term "Fv" generally refers to the smallest antibody fragment containing an intact antigen recognition and binding site. In some cases, the fragment can be composed of a heavy chain variable region and a light chain variable region as a tightly non-covalently bound dimer. The term "dsFv" generally refers to a disulfide bond-stablized Fv fragment, of which the bond between an individual light chain variable region and an individual heavy chain variable region is a disulfide bond. The term "dAb fragment" generally refers to an antibody fragment consisting of a VH domain. In the present application, the term "scFv" generally refers to a monovalent molecule formed by pairing a heavy chain variable domain and a light chainvariable domain of an antibody through covalent linkage by a flexible peptide linker. Such scFv molecules may have a general structure of NH₂-VL-linker-VH-COOH or NH₂-VH-linker-VL-COOH.

In the present application, the term "variable region" or "variable domain" generally refers to a domain of the heavy chain or the light chain of an antibody that is involved in the binding of the antibody to the antigen. In the present application, the term "variable" generally means that, there are strong variations in some portions of the sequence of the variable domain of an antibody, thus forming the binding and specificity of various specific antibodies for their particular antigens. The variability is not evenly distributed throughout the variable region of the antibody, and it is concentrated in three segments in the light chain variable region and the heavy chain variable region, which are referred to as complementary determining regions (CDR) or hypervariable regions (HVR), i.e., LCDR1, LCDR2, LCDR3, HCDR1, HCDR2 and HCDR3, respectively. The more highly conserved portions in the variable domain are referred to as framework regions (FR). The variable domain of a natural heavy chain and light chain each comprises four FR regions (H-FR1, H-FR2, H-FR3, H-FR4, L-FR1, L-FR2, L-FR3, L-FR4), most of which are of a β-folded conformation and linked by three CDR structural loop regions. The CDRs in each chain are held closely together by the FR regions and form the antigen binding sites of the antibody together with the CDRs from the other chain.

In the art, a variety of methods can be used to encode the variable regions of an antibody or to divide the CDRs of an antibody, e.g., Kabat numbering scheme and definition rules based on sequence variability (see, Kabat et, al., Protein Sequences in Immunology, 5th edition, National Institutes of Health, Bethesda, Maryland (1991)), Chothia numbering scheme and definition rules based on the location of structural loop regions (see, A1-Lazikani et, al., J Mol Biol 273:927-48,1997), IMGT numbering scheme and definition rules based on the amino acid sequence comparison of germ line V genes to efranc et, al., and Honneger's numbering scheme (AHo's), Martin numbering scheme, Gelfand numbering scheme, etc., see Mathieu Dondelinger et, al., Understanding the Significance and Implications of Antibody Numbering and Antigen-Binding Surface/Residue Definition, Front. Immunol., 16 October 2018.

In the present application, the term "monoclonal antibody" generally refers to antibodies obtained from a population of substantially homogeneous antibodies, that is, the individual antibodies that make up the population are identical, except for possible naturally occurring mutations and/or post-translational modifications (e.g., isomerization, amidation) that may exist in trace amounts. Monoclonal antibodies are highly specific and are directed against a single antigenic site.

In the present application, the term "chimeric antibody" generally refers to an antibody of which the variable region is derived from one species and the constant region is derived from another species. Generally, the variable region is derived from an antibody ("parental antibody") of a laboratory animal such as a rodent, and the constant region is derived from a human antibody, so that the resulting chimeric antibody is less likely to elicit an adverse immune response in human individuals compared to the parental (e.g., mouse-derived) antibodies.

In the present application, the term "humanized antibody" generally refers to an antibody in which some or all of the amino acids other than the CDR region of a non-human antibody (e.g., a mouse antibody) are replaced with corresponding amino acids derived from human immunoglobulins. In CDR regions, additions, deletions, insertions, substitutions, or modifications of amino acids are also permissible as long as they still retain the ability of the antibody to bind a specific antigen. Humanized antibodies may optionally comprise at least a portion of the constant region of human immunoglobulin. "Humanized antibodies" retain the antigen specificity similar to that of original antibodies. "Humanized" forms of non-human (e.g., mouse) antibody may minimally comprise chimeric antibodies derived from the sequences of non-human immunoglobulins. In some cases, the CDR region residues in human immunoglobulins (receptor antibodies) can be replaced with the CDR region residues of non-human species (donor antibodies) (such as, mice, rats, rabbits, or non-human primates) having desired properties, affinity and/or abilities. In some cases, the FR region residues in human immunoglobulins can be replaced with corresponding non-human residues. In addition, humanized antibodies may comprise amino acid modifications that are not present in the receptor antibodies or donor antibodies.

In the present application, the term "full human antibody" generally refers to an antibody of which all the portions (comprising the variable regions and the constant regions of the antibody) are encoded by human-derived genes. Methods for obtaining full human antibodies in the art may comprise phage display technology, transgenic mice technology, ribosome display technology, RNA-polypeptide technology, etc.

In the present application, the term "binding", "specific binding" or "being specific to" generally refers to measurable and reproducible interactions, such as the binding between an antigen and an antibody, which can determine the presence of a target in the presence of a heterogeneous population of molecules (comprising biological molecules). For example, an antibody binds to an epitope through its antigen binding domain, and the binding requires some complementarity between the antigen binding domain and the epitope. For example, an antibody that specifically binds a target (which may be an epitope) is an antibody that binds this target with greater affinity, avidity, more readily and/or for a longer duration than it binds other targets. When an antibody is more likely to bind to an epitope through its antigen binding domain than it would bind to random and unrelated epitopes, the antibody is referred to as "specifically binding" to that antigen.

In the present application, the term "polypeptide" or "protein" can be used interchangeably and generally refers to a polymer of amino acid residues. This term also applies to amino acid polymers in which one or more amino acid residues are analogs or mimics of corresponding naturally occurring amino acids, as well as naturally occurring amino acid polymers. Tis term may also comprise modified amino acid polymers, for example, glycoproteins formed by adding sugar residues or modification by phosphorylation. Polypeptide and protein can be produced from naturally occurring and non-recombinant cells or genetically engineered or recombinant cells and may comprise molecules with amino acid sequences of natural protein, or molecules with deletions, additions and/or substitutions of one or more amino acids of natural sequences. The term "polypeptide" and "protein" particularly comprises sequences with deletions, additions and/or substitutions of one or more amino acids of the antigen binding protein of the present application.

In the present application, the term "homologue" generally refers to an amino acid sequence or a nucleotide sequence having a certain homology with a wild-type amino acid sequence and a wild-type nucleotide sequence. The term "homology" may be equivalent to the "identity" of sequences. Homologous sequences may comprise amino acid sequences that are at least 80%, 85%, 90%, 99.1%, 99.2%, 99.3%, 99.4%, 99.5%, 99.6%, 99.7%, 99.8% or 99.9% the same as the subject sequence. In general, homologues will contain the same active sites as the subject amino acid sequence, and the like. Homology may be considered on the basis of similarity (i.e., amino acid residues having similar chemical properties/functions), or homology can be expressed in terms of the sequence identity. In the present application, a sequence having a percentage identity in either of the SEQ ID NOs of the mentioned amino acid sequences or nucleotide sequences refers to a sequence having the percentage identity over the whole length of the mentioned SEQ ID NOs.

In the present application, the term "isolated" generally refers to biological materials (e.g., viruses, nucleic acids or proteins) that are substantially free of components that are normally accompanied with or interact with them in their natural environment. The isolated biological materials optionally comprise additional materials that are not found in the biological materials in their natural environment (e.g., nucleic acids or proteins). In the present application, when referring to a protein, "isolated" generally means that the molecule is isolated and separated from the entire organism in which the molecule is naturally found, or that there are essentially no other biological macromolecules of the same type. When referring to a nucleic acid molecule, the nucleic acid molecule is entirely or partially isolated from the sequences to which it binds naturally, or the nucleic acid has heterologous sequences to which it binds, or the nucleic acid is isolated from chromosomes.

In the present application, the term "immunoconjugate" generally refers to a substance formed by linking an antigen-binding protein with other active agents, which may be small molecule active agents such as chemotherapeutic agents, toxins, immunotherapeutic agents, imaging probes, or spectroscopic probes.

In the present application, the term "nucleic acid" molecules generally refer to isolated nucleotides, deoxyribonucleotides or ribonucleotides of any length, or analogues thereof isolated from its natural environment or synthesized artificially.

In the present application, the term "vector" generally refers to a nucleic acid molecule capable of self-replication in a suitable host, which transfers the inserted nucleic acid molecule into and/or between host cells. The vector may comprise a vector mainly used for inserting DNA or RNA into cells, a vector mainly used for replicating DNA or RNA, and a vector mainly used for expression of DNA or RNA transcription and/or translation. The vector also comprises a vector with a variety of the above functions. The vector may be a polynucleotide that can be transcribed and translated into a polypeptide when introduced into a suitable host cell. Generally, by culturing a suitable host cell comprising the vector, the vector can produce desired expression products.

In the present application, the term "cell" generally refers to an individual cell, cell line, or cell culture that can comprise or has comprised a plasmid or vector comprising the nucleic acid molecule of the present application, or can express the antigen binding protein of the present application. The cells can comprise the progeny of a single cell. Due to natural, accidental or intentional mutations, the progeny cells may not necessarily be exactly the same as the original parent cells in terms of morphology or genome, as long as they can express the antibody or the antigen-binding fragment thereof of the present application. The cells can be obtained by transfecting cells *in vitro* with the vector of the present application. The cells may be prokaryotic cells (e.g., *Escherichia coli*) or eukaryotic cells (e.g., yeast cells, e.g., COS cells, Chinese Hamster Ovary (CHO) cells, HeLa cells, HEK293 cells, COS-1 cells, NS0 cells, or myeloma cells). In some cases, the cells may be mammalian cells. For example, the mammalian cells may be CHO-K1 cells.

In the present application, the term "pharmaceutical composition" generally refers to a preparation that exists in a form that allows the biological activity of the active ingredients to be effective and does not contain additional ingredients that would have unacceptable toxicity to the subject to whom the composition is to be administered.

In the present application, the term "treatment" generally refers to clinical interventions that are intended to alter the natural course of a disease in an individual being treated and that may be undertaken to achieve prevention or treatment or during a clinical condition. Desirable therapeutic effects comprise, but not limited to, preventing the onset or recurrence of a disease, alleviating symptoms, attenuating any direct or indirect pathological consequences of the disease, preventing metastasis, decreasing the progression rate of the disease, ameliorating or alleviating the state of the disease, and moderating or improving the prognosis. In some instances, the antigen binding protein (e.g., anti-PD-L1 antibody) of the present application can be used to delay the development of a disease or slow down the progression of the disease.

In the present application, the term "administration" generally refers to a method for giving a subject (e.g., a patient) a certain dose of compounds (e.g., anti-cancer therapeutic agents) or pharmaceutical compositions (e.g., a pharmaceutical composition comprising an anti-cancer therapeutic agent). The administration can be via any suitable routes, comprising parenteral, intrapulmonary and intranasal, and (if needed by local treatment) intralesional administration. Parenteral infusion comprises, e.g., intramuscular, intravenous, intraarterial, intraperitoneal or subcutaneous administration.

In the present application, the term "tumor" generally refers to all the neoplastic cell growth and proliferation (no matter malignant or benign) as well as all the precancerous and cancerous cells and tissues. In the present application, the tumor may be a tumor with high expression of PD-1 or PD-L1 in cells and tissues. The tumor may comprise a solid tumor and/or a non-solid tumor.

In the present application, the term "comprise" generally refers to the meaning of including, encompassing, containing, or embracing. In some cases, it also means "is/are" or "be composed of...".

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### Detailed Description of the Invention

In the present application, mice were immunized four times with recombinantly expressed human PD-L1 protein as the antigen, and mouse spleen cells with high serum titer were isolated and fused with SP2/0 myeloma cells. Monoclonal hybridoma cell lines expressing antibodies that specifically bind PD-L1 antigen were screened through techniques such as enzyme linked immunosorbent assay (ELSIA), fluorescence activated cell sorter (FACS), biomacromolecule interaction surface plasmon resonance (SPR) affinity assay and limited dilution cloning for monoclonal antibodies. The RNA of the candidate cell lines was then extracted, reverse transcribed into cDNA, and amplified by primer PCR to obtain the genes of the antibody heavy and light chain variable regions, which were then sequenced to obtain the gene sequences. After then, homology modeling and database comparison were employed to mutate the original murine sequence into a human sequence, resulting in a highly humanized, biologically active, and minimally immunogenic PD-L1 antigen-binding protein. Subsequently, homology modeling and database comparison were employed to mutate the original murine sequence into a humanized sequence, resulting in a highly humanized, biologically active, and low-immunogenic PD-L1 antigen binding protein.

### PD-L1 antigen binding protein

In one aspect, the present application provides an isolated antigen binding protein, which has one or more of the following properties:
(1) capable of binding to a PD-L1 protein at an EC₅₀ value of 0.07 µg/ml or above;
(2) capable of blocking the binding of the PD-L1 protein to a PD-1 protein;
(3) capable of promoting the activation of a T cell; and
(4) capable of inhibiting the volume increase of a tumor in the body.

In the present application, the binding affinity for PD-L1 protein can be determined by any methods known in the art. For example, the binding affinity can be determined by surface plasmon resonance (SPR), enzyme-linked immunosorbent assay (ELISA), binding antigen precipitation, equilibrium dialysis, biolayer interferometry (BLI) assay.

In the present application, the isolated antigen binding protein can bind to a PD-L1 protein at an EC₅₀ value of 0.07 µg/ml or above. For example, the EC₅₀ value may be about 0.075 µg/ml or above, about 0.080 µg/ml or above, about 0.085 µg/ml or above, about 0.090 µg/ml or above, about 0.095 µg/ml or above, about 0.1 µg/ml or above, about 0.2 µg/ml or above, about 0.5 µg/ml or above or about 1.0 µg/ml or above.

In the present application, the isolated antigen binding protein can bind to a PD-L1 protein at a KD value of 8×10⁻¹¹M or below. For example, the isolated antigen binding protein can bind to a human-derived PD-L1 at a KD value of about 7.5×10⁻¹¹M or below, about 7×10⁻¹¹M or below, about 6_{×}10⁻¹¹M or below, about 5×10⁻¹¹M or below, about 4×10⁻¹¹M or below, about 3×10⁻¹¹M or below, about 2×10⁻¹¹M or below, about 1×10⁻⁻¹¹M or below, about 5×10⁻¹²M or below, about 1×10⁻¹²M or below, for example, as detected by a FortieBio Octet molecule interaction analyzer.

In the present application, the isolated antigen binding protein can block the binding of PD-1 to PD-L1. For example, the blocking of the antigen binding protein on the binding of PD-1 to PD-L1 can be determined by fluorescence-activated cell sorting (FACS) or enzyme-linked immunosorbent assay (ELISA).

For example, host cells (e.g., CHO-K1 cells) stably expressing human PD-L1 are first incubated with decreasing amounts of unlabeled antigen binding protein, and subsequently incubated with biotin-labeled PD-1 protein. The cells are then analyzed by FACS to confirm that the antigen binding protein blocks the binding of PD-1 to PD-L1. For example, between about 0.001 µg/ml and about 100 µg/ml, between about 0.001 µg/ml and about 10 µg/ml, between about 0.01 µg/ml and about 1 µg/ml, between about 0.01 µg/ml and about 1 µg/ml, between about 0.001 µg/ml and about 0.1 µg/ml.

In the present application, the isolated antigen binding protein can promote the activation of a T cell. For example, the antigen binding protein can activate T cells that can stably express human PD-1 (e.g., Jurkat T cells that can stably express human PD-1 and NFAT-induced luciferase) in the presence of target cells that can express PD-L1 (for example, the target cells can stably express human PD-L1, e.g., the target cells may be CHO-K1-OS8-PD-L1-8D6 cells). The activation of T cells can be obtained by detecting the full-wavelength readings of the T cells on a microplate reader.

In the present application, the isolated antigen binding protein may comprise an antibody or an antigen binding fragment thereof.

In the present application, the antigen binding fragment may comprise Fab, Fab', F(ab)2, an Fv fragment, F(ab')2, scFv, di-scFv, VHH and/or dAb.

In the present application, the antibody is selected from the group consisting of a monoclonal antibody, a chimeric antibody, a humanized antibody, and a full human antibody.

In the present application, the isolated antigen binding protein competes with a reference antibody for binding to the PD-L1 protein, wherein the reference antibody may comprise a heavy chain variable region VH and a light chain variable region VL, the VH of the reference antibody may comprise a HCDR1, a HCDR2 and a HCDR3, the VL of the reference antibody may comprise a LCDR1, a LCDR2 and a LCDR3, and the HCDR1 of the reference antibody may comprise an amino acid sequence as set forth in SEQ ID NO: 1, the HCDR2 of the reference antibody may comprise an amino acid sequence as set forth in SEQ ID NO: 22, the HCDR3 of the reference antibody may comprise an amino acid sequence as set forth in SEQ ID NO: 23, the LCDR1 of the reference antibody may comprise an amino acid sequence as set forth in SEQ ID NO: 6, the LCDR2 of the reference antibody may comprise an amino acid sequence as set forth in SEQ ID NO: 23, and the LCDR3 of the reference antibody may comprise an amino acid sequence as set forth in SEQ ID NO: 8.

In the present application, the antigen binding protein may compete with the reference antibody for binding to the PD-L1 protein, that is, the antigen binding protein may compete with the reference antibody for binding to at least some of the same PD-L1 epitopes.

In the present application, the CDR sequences are all divided according to specific CDR division standard (e.g., Kabat method). It should be noted that if the CDR sequences obtained by dividing the amino acid sequences of a PD-L1 antigen binding protein according to specific CDR division standard (e.g., Kabat method) are identical to those involved in the present application, the amino acid sequences of the PD-L1 antigen binding protein are also within the scope of protection of the present application. The precise identification of CDR positions may vary slightly according to different CDR division standards, and therefore not only the CDRs set forth in the sequence listing but also the CDRs comprised within the VH and VL domains using other numbering systems are comprised in the present application. In addition, these VH and VL sequences can be used in a form of scFv or Fab.

In the present application, the PD-L1 antigen binding protein has at least one of HCDRs 1-3 as divided in any form of CDR division, which are comprised in the heavy chain variable region as set forth in any one of SEQ ID NOs. 11-16.

In the present application, the PD-L1 antigen binding protein has at least one of LCDRs 1-3 as divided in any form of CDR division, which are comprised in the light chain variable region as set forth in any one of SEQ ID NOs. 17-20.

In the present application, the isolated antigen binding protein may comprise a HCDR1, and the HCDR1 may comprise an amino acid sequence as set forth in SEQ ID NO: 1.

In the present application, the isolated antigen binding protein may comprise a HCDR2, and the HCDR2 may comprise an amino acid sequence as set forth in SEQ ID NO: 21. The HCDR2 may comprise an amino acid sequence as set forth below: WIYLGSGNAKY X₁X₂KF X₃G (SEQ ID NO: 21), wherein X₁ may be N or S; X₂ may be E or Q; and X₃ may be K or Q.

In the present application, the isolated antigen binding protein may comprise a HCDR2, and the HCDR2 may comprise an amino acid sequence as set forth in SEQ ID NO: 2 or 4.

In the present application, the isolated antigen binding protein may comprise a HCDR3, and the HCDR3 may comprise an amino acid sequence as set forth in SEQ ID NO: 22. The HCDR3 may comprise an amino acid sequence as set forth below: X₁VGTYWYFDV (SEQ ID NO: 22), wherein, wherein X₁ may be C or S.

In the present application, the isolated antigen binding protein may comprise a HCDR3, and the HCDR3 may comprise an amino acid sequence as set forth in SEQ ID NO: 3 or 5.

In the present application, the isolated antigen binding protein may comprise a HCDR1, a HCDR2 and a HCDR3, wherein the HCDR1 may comprise an amino acid sequence as set forth in SEQ ID NO: 1, the HCDR2 may comprise an amino acid sequence as set forth in SEQ ID NO: 2, and the HCDR3 may comprise an amino acid sequence as set forth in SEQ ID NO: 3; or
wherein the HCDR1 may comprise an amino acid sequence as set forth in SEQ ID NO: 1, the HCDR2 may comprise an amino acid sequence as set forth in SEQ ID NO: 4, and the HCDR3 may comprise an amino acid sequence as set forth in SEQ ID NO: 3; or
wherein the HCDR1 may comprise an amino acid sequence as set forth in SEQ ID NO: 1, the HCDR2 may comprise an amino acid sequence as set forth in SEQ ID NO: 2, and the HCDR3 may comprise an amino acid sequence as set forth in SEQ ID NO: 5; or
wherein the HCDR1 may comprise an amino acid sequence as set forth in SEQ ID NO: 1, the HCDR2 may comprise an amino acid sequence as set forth in SEQ ID NO: 4, and the HCDR3 may comprise an amino acid sequence as set forth in SEQ ID NO: 5.

In the present application, the isolated antigen binding protein may comprise a H-FR1, a C-terminal of the H-FR1 is connected to an N-terminal of the HCDR1 directly or indirectly, and the H-FR1 may comprise an amino acid sequence as set forth in SEQ ID NO: 48. For example, the H-FR1 may comprise an amino acid sequence as set forth in any one of SEQ ID NOs: 24-27.

In the present application, the isolated antigen binding protein may comprise a H-FR2, the H-FR2 is located between the HCDR1 and the HCDR2, and the H-FR2 may comprise an amino acid sequence as set forth in SEQ ID NO: 49. For example, the H-FR2 may comprise an amino acid sequence as set forth in any one of SEQ ID NOs: 28-29.

In the present application, the isolated antigen binding protein may comprise a H-FR3, the H-FR3 is located between the HCDR2 and the HCDR3, and the H-FR3 may comprise an amino acid sequence as set forth in SEQ ID NO: 50. For example, the H-FR2 may comprise an amino acid sequence as set forth in any one of SEQ ID NOs: 30-33.

In the present application, the isolated antigen binding protein may comprise a H-FR4, a located N-terminal of the H-FR4 is connected to a C-terminal of the HCDR3, and the H-FR4 may comprise an amino acid sequence as set forth in SEQ ID NO: 54. For example, the H-FR2 may comprise an amino acid sequence as set forth in any one of SEQ ID NOs: 34-35.

In the present application, the H-FR1, H-FR2, H-FR3, and H-FR4 are selected from any one group of amino acid sequences below:
(1) H-FR1: SEQ ID NO: 24, H-FR2: SEQ ID NO: 28, H-FR3: SEQ ID NO: 30, and H-FR4: SEQ ID NO: 34; or
(2) H-FR1: SEQ ID NO: 25, H-FR2: SEQ ID NO: 29, H-FR3: SEQ ID NO: 31, and H-FR4: SEQ ID NO: 35; or
(3) H-FR1: SEQ ID NO: 26, H-FR2: SEQ ID NO: 29, H-FR3: SEQ ID NO: 32, and H-FR4: SEQ ID NO: 35; or
(4) H-FR1: SEQ ID NO: 27, H-FR2: SEQ ID NO: 29, H-FR3: SEQ ID NO: 33, and H-FR4: SEQ ID NO: 35.

In the present application, the isolated antigen binding protein may comprise a heavy chain variable region VH, and the VH may comprise an amino acid sequence as set forth in any one of SEQ ID NOs: 11-16.

In the present application, the isolated antigen binding protein may comprise a heavy chain constant region, and the heavy chain constant region may be derived from IgG. For example, the heavy chain constant region may be derived from human IgG. The heavy chain constant region may be derived from IgG1, IgG2, IgG3, and IgG4. For example, the heavy chain constant region may be derived from human IgG1. For example, the human IgG1 constant region may comprise an amino acid sequence as set forth in SEQ ID NO: 55.

In the present application, the isolated antigen binding protein may comprise a LCDR1, and the LCDR1 may comprise an amino acid sequence as set forth in SEQ ID NO: 6.

In the present application, the isolated antigen binding protein may comprise a LCDR2, and the LCDR2 may comprise an amino acid sequence as set forth in SEQ ID NO: 23. The LCDR3 may comprise an amino acid sequence as set forth below: DTS X₁ X₂ X₃S (SEQ ID NO: 23), wherein X₁ may be N or S; X₂ may be L or V; and X₁ may be A or Q.

In the present application, the isolated antigen binding protein may comprise a LCDR2, and the LCDR2 may comprise an amino acid sequence as set forth in SEQ ID NO: 7, 9 or 10.

In the present application, the isolated antigen binding protein may comprise a LCDR3, and the LCDR3 may comprise an amino acid sequence as set forth in SEQ ID NO: 8.

In the present application, the isolated antigen binding protein may comprise a LCDR1, a LCDR2 and a LCDR3, wherein the LCDR1 may comprise an amino acid sequence as set forth in SEQ ID NO: 6, the LCDR2 may comprise an amino acid sequence as set forth in SEQ ID NO: 7, and the LCDR3 may comprise an amino acid sequence as set forth in SEQ ID NO: 8; or
wherein the LCDR1 may comprise an amino acid sequence as set forth in SEQ ID NO: 6, the LCDR2 may comprise an amino acid sequence as set forth in SEQ ID NO: 9, and the LCDR3 may comprise an amino acid sequence as set forth in SEQ ID NO: 8; or
wherein the LCDR1 may comprise an amino acid sequence as set forth in SEQ ID NO: 6, the LCDR2 may comprise an amino acid sequence as set forth in SEQ ID NO: 10, and the LCDR3 may comprise an amino acid sequence as set forth in SEQ ID NO: 8.

In the present application, the isolated antigen binding protein may comprise a LCDR1, a LCDR2 and a LCDR3 as well as a HCDR1, a HCDR2 and a HCDR3, wherein,
the HCDR1 may comprise an amino acid sequence as set forth in SEQ ID NO: 1, the HCDR2 may comprise an amino acid sequence as set forth in SEQ ID NO: 2, and the HCDR3 may comprise an amino acid sequence as set forth in SEQ ID NO: 3, and, the LCDR1 may comprise an amino acid sequence as set forth in SEQ ID NO: 6, the LCDR2 may comprise an amino acid sequence as set forth in SEQ ID NO: 7, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 4, and the HCDR3 may comprise an amino acid sequence as set forth in SEQ ID NO: 3, and, the LCDR1 may comprise an amino acid sequence as set forth in SEQ ID NO: 6, the LCDR2 may comprise an amino acid sequence as set forth in SEQ ID NO: 7, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 2, and the HCDR3 may comprise an amino acid sequence as set forth in SEQ ID NO: 5, and, the LCDR1 may comprise an amino acid sequence as set forth in SEQ ID NO: 6, the LCDR2 may comprise an amino acid sequence as set forth in SEQ ID NO: 7, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 4, and the HCDR3 may comprise an amino acid sequence as set forth in SEQ ID NO: 5, and, the LCDR1 may comprise an amino acid sequence as set forth in SEQ ID NO: 6, the LCDR2 may comprise an amino acid sequence as set forth in SEQ ID NO: 7, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 2, and the HCDR3 may comprise an amino acid sequence as set forth in SEQ ID NO: 3, and, the LCDR1 may comprise an amino acid sequence as set forth in SEQ ID NO: 6, the LCDR2 may comprise an amino acid sequence as set forth in SEQ ID NO: 9, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 4, and the HCDR3 may comprise an amino acid sequence as set forth in SEQ ID NO: 3, and, the LCDR1 may comprise an amino acid sequence as set forth in SEQ ID NO: 6, the LCDR2 may comprise an amino acid sequence as set forth in SEQ ID NO: 9, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 2, and the HCDR3 may comprise an amino acid sequence as set forth in SEQ ID NO: 5, and, the LCDR1 may comprise an amino acid sequence as set forth in SEQ ID NO: 6, the LCDR2 may comprise an amino acid sequence as set forth in SEQ ID NO: 9, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 4, and the HCDR3 may comprise an amino acid sequence as set forth in SEQ ID NO: 5, and, the LCDR1 may comprise an amino acid sequence as set forth in SEQ ID NO: 6, the LCDR2 may comprise an amino acid sequence as set forth in SEQ ID NO: 9, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 4, and the HCDR3 may comprise an amino acid sequence as set forth in SEQ ID NO: 3, and, the LCDR1 may comprise an amino acid sequence as set forth in SEQ ID NO: 6, the LCDR2 may comprise an amino acid sequence as set forth in SEQ ID NO: 10, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 2, and the HCDR3 may comprise an amino acid sequence as set forth in SEQ ID NO: 5, and, the LCDR1 may comprise an amino acid sequence as set forth in SEQ ID NO: 6, the LCDR2 may comprise an amino acid sequence as set forth in SEQ ID NO: 10, and the LCDR3 may comprise an amino acid sequence as set forth in SEQ ID NO: 8; or
the HCDR1 may comprise an amino acid sequence as set forth in SEQ ID NO: 1, the HCDR2 may comprise an amino acid sequence as set forth in SEQ ID NO: 4, and the HCDR3 may comprise an amino acid sequence as set forth in SEQ ID NO: 5, and, the LCDR1 may comprise an amino acid sequence as set forth in SEQ ID NO: 6, the LCDR2 may comprise an amino acid sequence as set forth in SEQ ID NO: 10, and the LCDR3 may comprise an amino acid sequence as set forth in SEQ ID NO: 8.

In the present application, the isolated antigen binding protein may comprise a L-FR1, a C-terminal of the L-FR1 is connected to an N-terminal of the LCDR1 directly or indirectly, and the L-FR1 may comprise an amino acid sequence as set forth in any one of SEQ ID NOs: 36-38.

In the present application, the isolated antigen binding protein may comprise a L-FR2, the L-FR2 is located between the LCDR1 and the LCDR2, and the L-FR2 may comprise an amino acid sequence as set forth in SEQ ID NO: 51. For example, the L-FR2 may comprise an amino acid sequence as set forth in any one of SEQ ID NOs: 39-41.

In the present application, the isolated antigen binding protein may comprise a L-FR3, the L-FR3 is located between the LCDR2 and the LCDR3, and the L-FR3 may comprise an amino acid sequence as set forth in SEQ ID NO: 52. For example, the L-FR2 may comprise an amino acid sequence as set forth in any one of SEQ ID NOs: 42-45.

In the present application, the isolated antigen binding protein may comprise a L-FR4, a located N-terminal of the L-FR4 is connected to a C-terminal of the LCDR3, and the L-FR4 may comprise an amino acid sequence as set forth in SEQ ID NO: 53. For example, the L-FR2 may comprise an amino acid sequence as set forth in any one of SEQ ID NOs: 46-47.

In the present application, the L-FR1, L-FR2, L-FR3, and L-FR4 are selected from any one group of amino acid sequences below:
(1) L-FR1: SEQ ID NO: 36, L-FR2: SEQ ID NO: 39, L-FR3: SEQ ID NO: 42, and L-FR4: SEQ ID NO: 46; or
(2) L-FR1: SEQ ID NO: 37, L-FR2: SEQ ID NO: 40, L-FR3: SEQ ID NO: 43, and L-FR4: SEQ ID NO: 47; or
(3) L-FR1: SEQ ID NO: 38, L-FR2: SEQ ID NO: 40, L-FR3: SEQ ID NO: 44, and L-FR4: SEQ ID NO: 47; or
(4) L-FR1: SEQ ID NO: 38, L-FR2: SEQ ID NO: 41, L-FR3: SEQ ID NO: 45, and L-FR4: SEQ ID NO: 47.

In the present application, the isolated antigen binding protein may comprise a light chain variable region VL, wherein the VL may comprise an amino acid sequence as set forth in any one of SEQ ID NOs: 17-20.

In the present application, the isolated antigen binding protein may comprise a light chain constant region, the light chain constant region may be derived from a light chain λ and a light chain κ. For example, the light chain constant region may comprise an amino acid sequence as set forth in SEQ ID NO: 56.

In the present application, the isolated antigen binding protein may comprise a heavy chain variable region VH and a light chain variable region VL, wherein,
the VH may comprise an amino acid sequence as set forth in SEQ ID NO: 11, and the VL may comprise an amino acid sequence as set forth in SEQ ID NO: 17, or
the VH may comprise an amino acid sequence as set forth in SEQ ID NO: 12, and the VL may comprise an amino acid sequence as set forth in SEQ ID NO: 18, or
the VH may comprise an amino acid sequence as set forth in SEQ ID NO: 13, and the VL may comprise an amino acid sequence as set forth in SEQ ID NO: 18, or
the VH may comprise an amino acid sequence as set forth in SEQ ID NO: 14, and the VL may comprise an amino acid sequence as set forth in SEQ ID NO: 18, or
the VH may comprise an amino acid sequence as set forth in SEQ ID NO: 15, and the VL may comprise an amino acid sequence as set forth in SEQ ID NO: 18, or
the VH may comprise an amino acid sequence as set forth in SEQ ID NO: 16, and the VL may comprise an amino acid sequence as set forth in SEQ ID NO: 18, or
the VH may comprise an amino acid sequence as set forth in SEQ ID NO: 13, and the VL may comprise an amino acid sequence as set forth in SEQ ID NO: 19, or
the VH may comprise an amino acid sequence as set forth in SEQ ID NO: 14, and the VL may comprise an amino acid sequence as set forth in SEQ ID NO: 19, or
the VH may comprise an amino acid sequence as set forth in SEQ ID NO: 15, and the VL may comprise an amino acid sequence as set forth in SEQ ID NO: 19, or
the VH may comprise an amino acid sequence as set forth in SEQ ID NO: 16, and the VL may comprise an amino acid sequence as set forth in SEQ ID NO: 19, or
the VH may comprise an amino acid sequence as set forth in SEQ ID NO: 14, and the VL may comprise an amino acid sequence as set forth in SEQ ID NO: 20, or
the VH may comprise an amino acid sequence as set forth in SEQ ID NO: 15, and the VL may comprise an amino acid sequence as set forth in SEQ ID NO: 20, or
the VH may comprise an amino acid sequence as set forth in SEQ ID NO: 16, and the VL may comprise an amino acid sequence as set forth in SEQ ID NO: 20.

In the present application, the CDRs of the antigen binding protein can be divided according to naming rules. For example, they can be divided according to Kabat rules.

In the present application, a part of each heavy chain or light chain amino acid sequence of the antigen binding protein is homogeneous to the corresponding amino acid sequence in an antibody derived from specific species or belongs to a certain class. For example, the variable region and constant portion of the light chain and the heavy chain are both derived from the variable region and constant region of an antibody of an animal species (such as, human). In the present application, the homologues may be proteins or polypeptides having at least about 85% (e.g., at least about 85%, about 90%, about 91%, about 92%, about 93%, about 94%, about 95%, about 96%, about 97%, about 98%, about 99% or above) sequence homology with the amino acid sequence of the protein and/or the polypeptide (for example, antibodies or fragments thereof specifically binding to a PD-L1 protein).

In the present application, the homology generally refers to the similarity, likeness or correlation between two or more sequences. The alignment for determining the percentage of sequence homology can be achieved in a variety of ways known in the art, for example, by using publicly available computer softwares, such as BLAST, BLAST-2, ALIGN or Megalign (DNASTAR) softwares. A person skilled in the art can determine appropriate parameters for aligning sequences, comprising any algorithms needed to achieve the maximum alignment over the full-length sequence range being compared or within the target sequence region. The homology can also be determined by the following methods: FASTA and BLAST. A description of FASTA algorithm can be found in "Improved tools for biological sequence comparison" to W. R. Pearson and D. J. Lipman, Proc. Natl. Acad. Sci., 85: 2444-2448, 1988; and "Rapid and Sensitive Protein Similarity Searches" to D. J. Lipman and W. R. Pearson, Science, 227: 1435-1441, 1989. A description of BLAST algorithm can be found in "Basic Local Alignment Search Tool" to S. Altschul, W. Gish, W. Miller, E. W. Myers, and D. Lipman, Journal of Molecular Biology, 215: 403-410, 1990.

### Detection method

The physical/chemical properties and/or biological activity of the PD-L1 antigen binding protein of the present application can be identified, screened or characterized by a variety of assays known in the art.

In one aspect, the antigen binding activity of the antigen binding protein of the present application can be tested by, for example, known methods such as enzyme-linked immunosorbent assay (ELISA), immunoblotting (e.g., Western blotting), fluorescence activated cell sorter (e.g., FACS), immunohistochemistry, and immunofluorescence.

### Nucleic acids, vectors, host cells and preparation method

In another aspect, the present application further provides one or more isolated nucleic acid molecules, which may encode the antigen binding protein of the present application. For example, each of the one or more nucleic acid molecules may encode an intact antigen binding protein or a part thereof (e.g., one or more of HCDRs 1-3, LCDRs 1-3, VL, VH, light chain or heavy chain).

The nucleic acid molecules of the present application may be isolated. For example, they can be produced or synthesized by the following methods: (i) amplification *in vitro,* e.g., being produced by amplification through polymerase chain reaction (PCR), (ii) being produced by cloning and recombination, (iii) being purified, for example by enzymatic digestion and gel electrophoresis fractionation, or (iv) being synthesized, for example through chemical synthesis. In some embodiments, the isolated nucleic acid are nucleic acid molecules prepared by a recombinant DNA technology.

In the present application, the nucleic acids encoding the antibody and the antigen binding fragment thereof can be prepared by a variety of methods known in the art, comprising but not limited to, overlap extension PCR using restrictive fragment operation or using synthetic oligonucleotide.

In another aspect, the present application provides one or more vectors, which comprise the one or more nucleic acid molecules of the present application. Each vector may comprise one or more of the nucleic acid molecules. In addition, the vector may also comprise other gene(s), e.g., a marker gene that allows the selection of the vector in an appropriate host cell and under appropriate conditions. Moreover, the vector may further comprise an expression control element that allows the coding region to be properly expressed in an appropriate host. For example, the vector is an expression vector.

In another aspect, the present application provides a host cell which may comprise one or more nucleic acid molecules of the present application and/or one or more vectors of the present application. In some embodiments, each kind of or each of the host cells may comprise one or one kind of the nucleic acid molecules or vectors of the present application. In some embodiments, each kind of or each of the host cells may comprise multiple (e.g., two or more) or multiple kinds of (e.g., two or more kinds of) the nucleic acid molecules or vectors of the present application.

In another aspect, the present application provides a method for preparing the antibody or the antigen binding fragment thereof. The method may comprise culturing the host cell of the present application under a condition enabling the expression of the antibody or the antigen binding fragment thereof. For example, the method can be performed by employing appropriate medium, appropriate temperature, and culturing time, etc., which are known to persons of ordinary skills in the art.

### Pharmaceutical composition, method, use

In another aspect, the present application provides a pharmaceutical composition, which may comprise the antigen binding protein, the polypeptide, the nucleic acid molecules, the vector, the host cell of the present application, and optionally a pharmaceutically acceptable carrier. The pharmaceutically acceptable adjuvant is non-toxic to the recipient at the dosage and concentration used, and the pharmaceutical composition of the present application may further comprise more than one active compounds, which are generally those having complementary activities that do not adversely affect each other. The type and effective dose of such drugs depend on the amount and type of the antagonist present in the preparation, as well as clinical parameters of the subject.

The pharmaceutical composition can be used to inhibit the tumor growth. For example, the pharmaceutical composition of the present application can inhibit or delay the development or progress of a disease, reduce the tumor size (even substantially eliminate the tumor), and/or alleviate and/or stabilize the disease state.

The pharmaceutical composition of the present application may comprise a prophylactically and/or therapeutically effective amount of the antibody or the antigen-binding fragment thereof. The prophylactically and/or therapeutically effective amount is a dose required to prevent and/or treat (at least partially treat) a disease or disorder and/or any complications thereof in a subject suffering from or having a risk of developing the disease or disorder.

In another aspect, the present application provides a use of the antigen binding protein and/or the fusion protein in the preparation of a drug for treating a cancer, inhibiting tumor growth and/or inhibiting proliferation of tumor cells. In some embodiments, the tumor or cancer is a tumor or cancer with abnormal expression of PD-L1. In the present application, the tumor may comprise a tumor with high expression of PD-1 or PD-L1. In the present application, the tumor may comprise a tumor with high expression of PD-L1. In the present application, the tumor may comprise a solid tumor. In the present application, the tumor may comprise colon cancer.

In another aspect, the present application provides a method for inhibiting the binding of PD-L1 to PD-1, which comprises administering the antigen binding protein and/or the polypeptide of the present application. For example, the method may be an *ex vivo* or *in vitro* method. For example, the method may be a non-therapeutic method. In some cases, the method may comprise contacting a biological sample with the antigen binding protein of the present application and/or PD-L1 under a condition allowing the binding of the antigen binding protein and/or PD-1 to PD-L1, to detect whether a complex is formed between the antigen binding protein and PD-L1, and whether a complex is formed between PD-1 and PD-L1.

In another aspect, the present application provides a method for promoting the activation of a T cell, which comprises administering the isolated antigen binding protein and/or the polypeptide. For example, the method may be an *ex vivo* or *in vitro* method. For example, the method may be a non-therapeutic method.

In another aspect, the present application provides a method for detecting the presence and/or content of PD-L1 protein, which comprises administering the isolated antigen binding protein and/or the polypeptide. For example, the method may be an ex vivo or in vitro method. For example, the method may be a non-therapeutic method.

The present application further provides a use of the antigen binding protein in the method for diagnosing a subject suffering from a tumor or cancer. The method comprises determining the presence or expression level of PD-L1 in a sample obtained from a subject by contacting the sample with the antigen binding protein of the present application and detecting the presence of the bound antibody.

In another aspect, the present application provides a chimeric antigen receptor (CAR), which may comprise the nucleic acid molecules of the present application or the antigen binding protein of the present application.

In another aspect, the present application provides an antibody-drug conjugate, which may comprise the antigen binding fragment.

In another aspect, the present application provides a kit, which may comprise the antigen binding protein of the present application, a chimeric antigen receptor, a genetically modified cell, an antibody-drug conjugate, and/or the pharmaceutical composition of the present application. The kit may comprise the antigen binding protein of the present application, the chimeric antigen receptor, the genetically modified cell, and/or the antibody-drug conjugate in a single common vessel, or may also be optionally combined with one or more therapeutic agents and optionally formulated in the pharmaceutical composition.

In another aspect, the present application provides a drug delivery device, which can be used to administer the antigen binding protein of the present application or the pharmaceutical composition thereof.

Without intending to be limited by any theory, the following examples are only to illustrate the antigen binding protein, preparation method and use of the present application, and not intended to limit the scope of the present application.

### EXAMPLES

### Example 1. Screening of Monoclonal Antibody

### 1.1 Immunization of Animals

On Day 1, the prime immunization was performed by subcutaneous multi-site injection of an antigen, a recombinant human PD-L1 protein (Item No.: 10084-H08H, Sino Biological Inc.) emulsified with the complete Freund's adjuvant (CFA) in an amount of 50 µg/mouse. Subsequently, the boost immunization was performed three times, and the mice were detected for titer of anti-PD-L1 antibody in their serum by ELISA method. Mice with high antibody titer in serum were selected for splenocyte fusion.

### 1.2 Fusion and Screening of Hybridoma

Spleen was taken from four mice with high titer for hybridoma fusion. The mouse spleen was ground into a cell suspension; the spleen lymphocytes were mixed with myeloma cells Sp2/0 after red blood cells were removed; the spleen lymphocytes were fused with the myeloma cells Sp2/0 by mediation with an electro-fusion instrument; and a HAT-containing medium was added for culture and screening to give hybridoma cells. The hybridoma cells were subject to primary screening by antigen-antibody indirect ELISA assay. 304 polyclonal cell strains were selected for monocloning by limited dilution method. After cell clones were generated, the culture supernatant of monoclonal strains was detected by ELISA method for their binding activity with human and *Macaca mulatta* PD-L1, and detected on a flow cytometer for their binding activity with PD-L1-expressing ES-2 cells. Strains with high binding activity were selected therefrom, and the respective culture supernatant was further subject to affinity detection using Biacore. Finally, 37 strains with high binding activity and high affinity were screened out for gene cloning of antibody variable regions.

### 1.3 Preparation of Anti-PD-L1 Human-Mouse Chimeric Antibody

37 cloned strains obtained by hybridoma screening were first used to extract the total RNA using the RNA Extraction Kit (Item No.: 74106, from QIAGEN) in accordance with the manufacturer's instructions, followed by cloning the cDNA sequences expressing the mouse antibody contained in the hybridoma cells using the 5' rapid amplification of cDNA ends (5' RACE) technology from Takara. Then, the antibody variable region sequences were amplified by two rounds of PCR, the PCR products were analyzed by agarose gel electrophoresis, and the variable region amplicons of both the heavy chain and the light chain have predicted size of about 500 base pairs. The amplified PCR product with appropriate band size obtained from the reaction was cloned into the vector pEASY-Blunt Simple vector (Item No.: CB111-02, TransGen Biotech), and transformed into Stellar *E. coli* competent cells (Item number: 636763, TAKARA). The clones were screened by colony PCR with universal M13 forward or reverse primers to select 2-3 clones from each reaction for DNA sequencing analysis. The result of respective sequencing reaction for each clone were analyzed using the Expasy-translate tool (http://web.expasy.org/translate/). Finally, 30 mouse monoclonal antibody variable region sequences were successfully extracted. The heavy chain and light chain variable region genes were amplified from the 30 correct clones by PCR, and linked to the vector containing the human IgG1 constant region sequence and the vector containing the human Igκ constant region sequence, respectively, to construct the expression vectors of both the heavy chain and the light chain of the chimeric antibody. Among them, the leucine at positions 234 and 235 (EU numbering system) of the heavy chain constant region CH2 was mutated into alanine to attenuate the functional activities mediated by the antibody Fc fragment, such as, ADCC/CDC/ADCP. The expression vectors were mixed with polyetherimide (PEI) to form a liposome complex, and then transfected into CHO-S cells (Item No.: R80007, Thermo Fisher Scientific (China) Co., Ltd.). After culture for 7 days, the antibodies were purified by protein A affinity chromatography column. The resultant chimeric antibodies were tested for their biological activities *in vitro* and *in vivo.*

### 1.4 Detection of PD-1/PD-L1 Blocking Activity of Anti-PD-L1 Human-Mouse Chimeric Antibodies

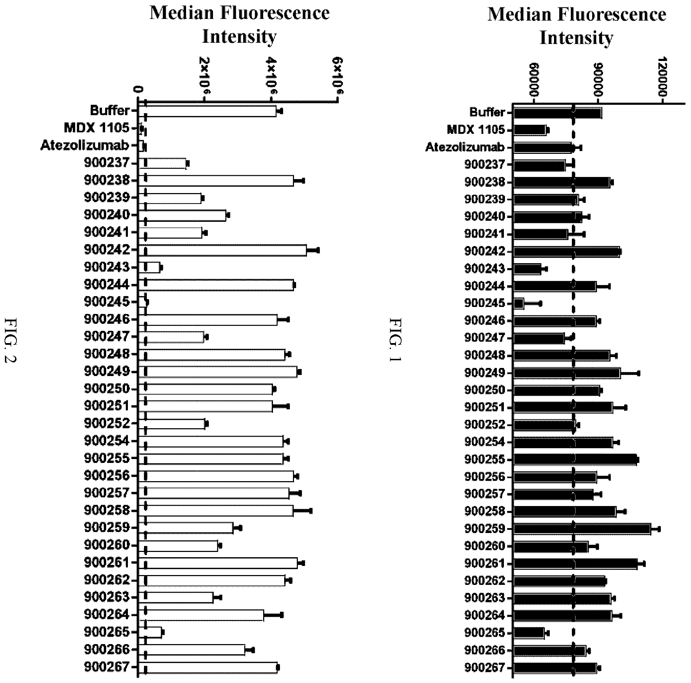

The blocking activity of the antibodies was detected in two aspects:
①The blocking effect of the antibodies on the binding of free PD-1 to cell surface PD-L1 was detected. The CHO-K1-huPD-L1 cells overexpressing human PD-L1 were plated in a 96-well plate at 2×10⁴/well. A human PD-1-his tag recombinant protein (Item No.: 10377-H08H-100, from Sino Biological Inc.) was diluted to 6 µg/mL with medium, added into the 96-well plate at 20 µl/well, mixed well, and incubated at room temperature for 30 min. Then, 20 µl of the diluted sample to be tested was added, mixed well, and incubated at room temperature for 30 min. The mixture was centrifuged at 300 g for 5 min, the supernatant was removed, and the cells were washed twice with FACS buffer. Anti-his-dylight 488 (Item No.: ab117512, Abcam) secondary antibodies were diluted at 1:500, added, mixed well, and incubated at room temperature for 15 min. The cells were washed three times and then detected on an instrument for their fluorescence intensity on the surface. The results are shown in FIG. 1.
②The blocking effect of the antibodies on the binding of free PD-L1 to cell surface PD-1 was detected. The CHO-K1-huPD-1 cells overexpressing human PD-1 were plated in a 96-well plate at 2×10⁴/well. A human PD-L1-moFc recombinant protein (SEQ ID NO. 57) was diluted to 15 µg/mL with medium, added into the 96-well plate at 20 µl/well, mixed well, and incubated at room temperature for 15 min. Then, 20 µl of the diluted sample to be tested was added, mixed well, and incubated at room temperature for 15 min. The mixture was centrifuged at 300 g for 5 min, the supernatant was removed, and the cells were washed twice with FACS buffer. Anti-moFc-alexa 488 (Item No.: 115-545-071, Jackson) secondary antibodies were diluted at 1:300, added, mixed well, and incubated at room temperature for 15 min. The cells were washed three times and detected on an instrument for their fluorescence intensity on the surface. The results are shown in FIG. 2.

In the blocking experiment, the lower the detection value, the better the blocking effect of the PD-L1 antibody. According to the results in FIGs. 1-2, it can be seen that the antibody 900245 has the highest blocking effect and has non-inferiority compared to the control antibody.

### 1.5 Detection of Affinity of Anti-PD-L1 Antibodies by SPR Method

The Anti-Human Capture-CM5 chips (Item No.: BR-1005-30, GE) were prepared according to the coupling method of the Human Antibody Captrue Kit (Item No.: BR-1008-39, GE) and the amino coupling kit (Item No.: BR-1000-50, GE). The chips were equilibrated at room temperature for 20-30 min and assembled into the Biacore 8K instrument. B5D1 was diluted to the experimental working concentration with the equilibrium buffer. The antigen was diluted to 50 nM with the equilibrium buffer, and then diluted to give samples with 7 concentration gradients by 3× serial dilution, and 2 samples with zero concentration (i.e. the equilibrium buffer) and a duplicate sample with one concentration (typically, a duplicate sample with the minimum concentration) were set. According to the antibody, the antigen huPD-L1 (Item No.: 10084-H08H, Sino Biological), and the regeneration order, the samples with 10 antigen concentrations (2 with zero concentration, 7 with gradient concentrations and 1 with duplicate concentration) were subject to experimental analysis with an antigen injection flow rate of 30 µl/min, the binding time of 120 seconds, and the dissociation time of 600 seconds. At the end of the analysis, a respective analysis program was used to analyze the data to confirm that there was no significant reference binding. The data were fitted with Kinetics, 1: 1 binding model, to obtain the kinetic-related parameters Ka, Kd, and KD values of the human-mouse chimeric antibody and the control antibody. The results are shown in Table 1. The chimeric antibody 900245 has a high affinity to the human PD-L1 antigen, and is superior to the control antibodies from other manufacturers (MDX 1105, Atezolizumab, Durvalumab and Avelumab, which are obtained by synthesis, gene expression, and purification according to the sequences disclosed by BMS, Roche, AstraZeneca, and Pfizer, respectively).

**Table 1 Measurement of Affinity of Chimeric Antibody to PD-L1**

| Sample Name | ka (1/Ms) | kd (1/s) | KD (M) |
|---|---|---|---|
| MDX 1105 | 5.649×10⁶ | 2.03×10⁻³ | 3.586×10⁻¹⁰ |
| Atezolizumab | 9.40×10⁵ | 1.72×10⁻⁴ | 1.83×10⁻¹⁰ |
| Durvalumab | 1.86×10⁶ | 4.58×10⁻⁴ | 2.46×10⁻¹⁰ |
| Avelumab | 7.48×10⁵ | 1.02×10⁻⁴ | 1.37×10⁻¹⁰ |
| 900245 | 1.235×10⁶ | 9.540×10⁻⁵ | 7.726×10⁻¹¹ |

### Example 2 Humanization of Antibodies

The variable regions of 900245 (VH: SEQ ID NO. 11; VL: SEQ ID NO. 17) were humanized to convert the mouse sequence into a human sequence. The original heavy chain mVH sequence of the antibody 900245 was designed into 5 humanized sequences (huVH1, huVH2, huVH3, huVH4, and huVH5), and the original light chain mVL sequence of the antibody 900245 was designed into 3 humanized sequences (huVL1, huVL2, huVL3). The 8 sequences designed were combined to form 12 pairs of humanized antibodies for subsequent expression verification. The mouse variable region sequence of 900245: the underlined part is the CDR region (Kabat definition).

Among them, the huVH1-5 have amino acid sequences as set forth in SEQ ID NO. 12, SEQ ID NO. 13, SEQ ID NO. 14, SEQ ID NO. 15, and SEQ ID NO. 16, respectively; and the huVL1-3 have amino acid sequences as set forth in SEQ ID NO. 18, SEQ ID NO. 19, and SEQ ID NO. 20, respectively.

The 5 humanized heavy chain variable region genes linked to the human IgG1 constant region gene (with amino acid sequence as set forth in SEQ ID NO. 55) and the 3 humanized light chain variable region genes linked to the human Igκ constant region gene (with amino acid sequence as set forth in SEQ ID NO. 56) were constructed into the pcDNA3.4 expression vector. The plasmid was transfected into ExpiCHO-s cells and transiently expressed. The protein was purified by Protein A, detected by ELISA and FACS for its binding activity and SPR for its affinity, and screened. The results are summarized in Table 2. Among them, 900245Ab-H1L1 is the humanized antibody obtained by linking VH as huVH1 and VL as huVL1 to the aforesaid constant regions, respectively.

**Table 2 Screening Data of Humanized antibodies**

| Sample Name | VH (SEQ ID NO.) | VL (SEQ ID NO.) | Degree of humanization | Expression quantity (µg/mL) | SDS-PAGE (%) | SEC (%) | ELISA EC50 | SPR KD |
|---|---|---|---|---|---|---|---|---|
| | | | | | | | (µg/ml) | (M) |
| 900245 | 11 | 17 | N/A | N/A | N/A | N/A | 0.499 | 7.20E-10 |
| 900245Ab-H1L1 | 12 | 18 | 95.90% | 86.8 | >95.0 | 93.86 | 0.3615 | 5.96E-10 |
| 900245Ab-H2L1 | 13 | 18 | 96.40% | 108.37 | >95.0 | 94.03 | 0.3845 | 6.39E-10 |
| 900245Ab-H3L1 | 14 | 18 | 97.90% | 127.77 | >95.0 | 95.37 | 0.3814 | 5.76E-10 |
| 900245Ab-H4L1 | 15 | 18 | 98.30% | 104.99 | >95.0 | 97.15 | 0.5893 | 7.26E-10 |
| 900245Ab-H5L1 | 16 | 18 | 98.30% | 97.1 | >95.0 | 95.28 | 0.5024 | 6.45E-10 |
| 900245Ab-H2L2 | 13 | 19 | 97.00% | 94.69 | >95.0 | 98.51 | 0.5137 | 8.16E-10 |
| 900245Ab-H3L2 | 14 | 19 | 98.50% | 104.91 | >95.0 | 98.59 | 0.7874 | 7.64E-10 |
| 900245Ab-H4L2 | 15 | 19 | 98.90% | 77.86 | >95.0 | 98.79 | 1.313 | 1.97E-09 |
| 900245Ab-H5L2 | 16 | 19 | 98.90% | 68.28 | >95.0 | 98.75 | 0.9906 | 1.67E-09 |
| 900245Ab-H3L3 | 14 | 20 | 98.90% | 130.51 | >95.0 | 98.89 | 8.699 | 1.04E-08 |
| 900245Ab-H4L3 | 15 | 20 | 99.40% | 91.22 | >95.0 | 99.11 | ∼ 1518 | 4.37E-08 |
| 900245Ab-H5L3 | 16 | 20 | 99.40% | 83.9 | >95.0 | 99.34 | ∼ 1407 | 3.09E-08 |

The results show that H5L1 (CoAD1445) has high binding activity and affinity, high degree of humanization, and optimal expression purity and expression quantity. The variable region genes of huVH5 and huVL1 were constructed into antibody expression vectors and transiently transfected into CHO-S cells. After culture for expression at 32°C for 7 days, the supernatant was collected by centrifugation and purified by Protein A affinity chromatography column to obtain the humanized anti-PD-L1 antibody No. 900760.

### Example 3 Measurement of In Vitro Property of Humanized Antibody

### 3.1 Binding to Cell Surface PD-L1 Antigen

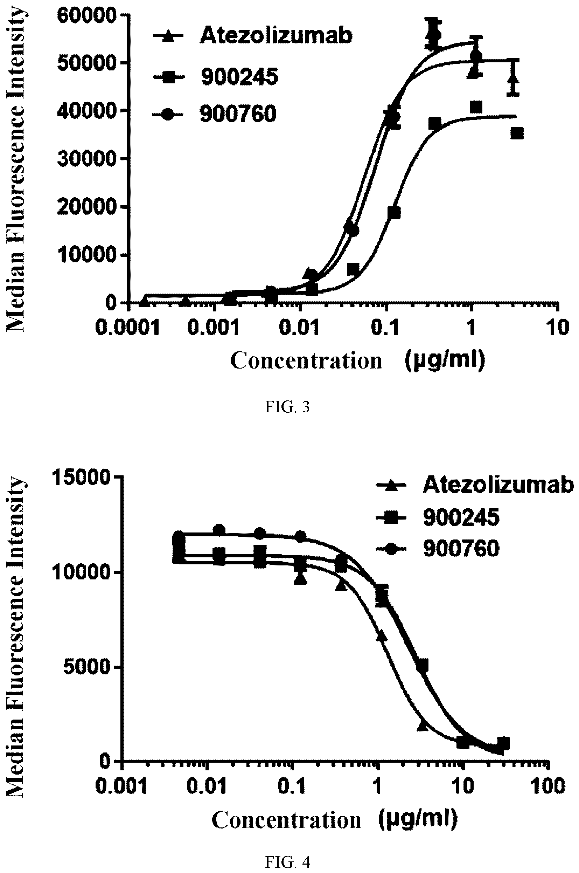

CHO-K1-huPD-L1 cells overexpressing human PD-L1 were plated in a 96-well plate at 2×10⁴/well, and the 3-fold serial gradient dilutions of the samples to be tested were added at 20 µl/well, and co-incubated with the cells at room temperature for 30 min. The cells were washed by centrifugation to remove unbound antibodies, and 20 µl of R-Phycoerythrin AffiniPure Goat Anti-Human IgG, Fcy Fragment Specific (Item No.: 109-115-098, Jackson ImmunoResearch; diluted with 1% (w/v) BSA-PBS at 1:200) fluorescent secondary antibodies were added to each well, and incubated for 15 min. The cells were then washed twice, resuspended in 100 µl of 1% (w/v) BSA-PBS, and detected for their fluorescence intensity at 585 nm by flow cytometry (BD, FACS Cantoll) under 488 nm excitation. The results are shown in FIG. 3. The EC₅₀ of the humanized antibody 900760 and the parent chimeric antibody are 0.075 µg/ml and 0.125 µg/ml, respectively, and the EC₅₀ of Roche's control antibody Atezolizumab is 0.056 µg/ml, indicating that the humanized antibody has an elevated binding activity to the antigen, which is close to that of the control antibody.

### 3.2 Detection of Affinity of Humanized Anti-PD-L1 Antibodies by SPR Method

The Anti-Human Capture-CM5 chips (Item No.: BR-1005-30, GE) were prepared according to the coupling method of the Human Antibody Captrue Kit (Item No.: BR-1008-39, GE) and the amino coupling kit (Item No.: BR-1000-50, GE). The chips were equilibrated at room temperature for 20-30 min and assembled into the Biacore 8K instrument. B5D1 was diluted to the experimental working concentration with the equilibrium buffer. The antigen was diluted to 50 nM with the equilibrium buffer, and then diluted to give samples with 7 concentration gradients by 3-fold serial dilution, and 2 samples with zero concentration (i.e. the equilibrium buffer) and a duplicate sample with one concentration (typically, a duplicate sample with the minimum concentration) were set. According to the antibody, the antigen huPD-L1 (Item No.: 10084-H08H, Sino Biological), and the regeneration order, the samples with 10 antigen concentrations (2 with zero concentration, 7 with gradient concentrations and 1 with duplicate concentration) were subject to experimental analysis with an antigen injection flow rate of 30 µl/min, the binding time of 120 seconds, and the dissociation time of 600 seconds. At the end of the analysis, a respective analysis program was used to analyze the data to confirm that there was no significant reference binding. The data were fitted with Kinetics, 1: 1 binding model, to obtain the kinetic-related parameters Ka, Kd, and KD values of the humanized antibody and the control antibody. The results are shown in Table 3. The results show that the humanized antibody 900760 has a high affinity for the human PD-L1 antigen, which is superior to that of the control antibody (Atezolizumab) from another manufacturer.

**Table 3 Measurement of Affinity of Humanized Antibody to PD-L1**

| Ligand | Analyte | ka (1/Ms) | kd (1/s) | KD (M) |
|---|---|---|---|---|
| 900245 | Human PD-L1 | 3.68E+05 | 1.27E-04 | 3.46E-10 |
| Atezolizumab | Human PD-L1 | 1.71E+05 | 3.29E-04 | 1.92E-09 |
| 900760 | Human PD-L1 | 3.45E+05 | 1.58E-04 | 4.59E-10 |
| | Cynomolgus PD-L1 | 1.98E+05 | 1.38E-04 | 6.95E-10 |
| | Rabbit PD-L1 | 1.67E+05 | 2.35E-03 | 1.40E-08 |
| | rat PD-L1 | NB | NB | NB |
| | Mouse PD-L1 | NB | NB | NB |

### 3.3 Effective Blocking of Interaction of PD-1/PD-L1

The blocking effect of the antibody on free PD-L1 to cell surface PD-1 was detected. The human PD-L1-moFc recombinant protein was diluted to 15 µg/ml with culture medium, and added to a 96-well plate at 20 µl/well. The antibodies to be tested were diluted with 3-fold gradient, added at 20 µL/well into the 96-well plate with the antigen added, mixed well, and incubated at room temperature for 15 min. CHO-K1-huPD-1 cells overexpressing human PD-1 were added at 2×10⁴/well into the 96-well plate pre-incubated with antigen-antibody, mixed well, and incubated at room temperature for 15 min. The mixture was centrifuged at 300 g for 5 min, the supernatant was removed, and the cells were washed twice with FACS buffer. Anti-moFc-Alexa 488 (Item No.: 115-545-071, Jackson) secondary antibodies were diluted at 1:300, added, mixed well, and incubated at room temperature for 15 min. The cells were washed three times and measured on instrument for the fluorescence intensity on their surfaces. The results are shown in FIG. 4. The IC₅₀ of the humanized antibody 900760 and the parent chimeric antibody are 2.272 µg/ml and 2.838 µg/ml, respectively, and the IC₅₀ of Roche's control antibody Atezolizumab is 1.311 µg/ml, indicating that the humanized antibody keeps the blocking activity on the PD-1/PD-L1 interaction well, which is close to that of the control antibody.

### 3.4 Promotion of T Cell Activation

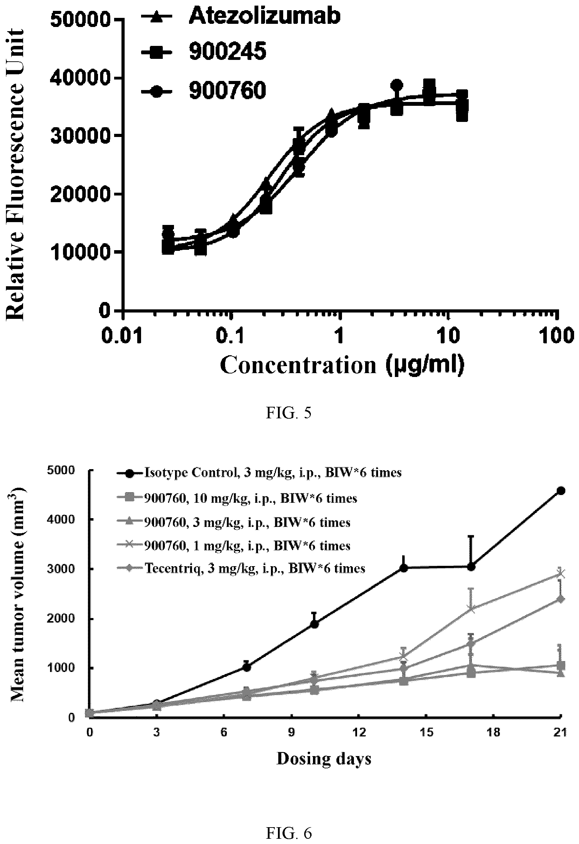

The detection system is composed of two genetically-engineered cell lines: Jurkat-NFAT-PD-1-5B8 cells (PD-1 effector cells), which are Jurkat T cells stably expressing human PD-1 and NFAT-induced luciferase; and CHO-K1-OS8-PD-L1-8D6 cells, which are used as target cells and can stably express human PD-L1 and T cell activating antibody OKT3 single chain antibody on the cell surface. The antibodies to be tested were diluted to 40 µg/ml with medium, and further subject to 2-fold gradient dilution for use. The effector cells Jurkat-NFAT-PD-1-5B8 were counted, resuspended to 5×10⁵/ml, and plated into a 96-well white-bottom plate at 30 µl/well. The target cells CHO-K1-OS8-PD-L1-8D6 were counted, resuspended at 5×10⁵/ml, and added at 30 µl/well. The diluted samples to be tested were added at 30 µl/well. After mixing well, the mixture was incubated in a CO₂ incubator at 37°C for 6 h. At the end of incubation, the plate was equilibrated at room temperature for at least 15 min. Then, the equilibrated Bio-Glo^{™} Luciferase Assay System reagent was added to the 96-well white plate at 90 µl/well, reacted at room temperature in dark for 5 min. Values were read using the MD SpectraMax^{®} i3x microplate reader at full wavelength. Using GraphPad Prism 7 software, a four-parameter equation fitting was carried out with RLU values vs antibody working concentrations to analyze the data. The results are shown in FIG. 5. The EC₅₀ of the humanized antibody 900760 and the parent chimeric antibody are 0.416 µg/ml and 0.287 µg/ml, respectively, and the EC₅₀ of Roche's control antibody Atezolizumab is 0.222 µg/ml, indicating that the humanized antibody of the present application can effectively promote the activation of T cells, with a comparable effect with the control antibody.

### Example 4 In Vivo Anti-Tumor Activity of Humanized Antibody

A transplanted tumor model of MC38 colon cancer cell line with humanized PD-L1 (MC38-hPD-L1) was constructed on PD-L1-humanized C57BL/6J mice (hPD-L1-C57BL/6J) for testing the *in vivo* anti-tumor efficacy of the anti-PD-L1 antibody. 6-8-week-old female mice were subcutaneously inoculated with MC38-hPD-L1 cell suspension on the right side of the mice. When the tumor volume was about 100 mm³, the mice were randomly grouped with 6 mice in each group, and started to be administered. Each group was administered intraperitoneally, twice a week for 3 consecutive weeks, with a total of 6 times. The animals were measured twice a week for their tumor diameter and body weight, observed for their living conditions, and recorded for any abnormal conditions. The tumor volume (TV) is calculated in accordance with the formula of: TV = 1/2×a×b², where: a represents the long diameter of the tumor; and b represents the short diameter of the tumor. The tumor volume inhibition rate (TGI) is calculated in accordance with the formula of: TGI = [1 - (TVt - TVinitial)/ (CVt - CVinitial)]×100%, where TVt represents the tumor volume of the treatment group at each measurement; TVinitial represents the tumor volume of the treatment group at the time of grouping for administration; CVt represents the tumor volume of the control group at each measurement; and CVinitial represents the tumor volume of the control group at the time of grouping for administration. Analysis and processing were performed based on the original data. The result analysis was expressed as the mean and standard error (Mean ± SEM). The difference in tumor volume between the control group and the administration group was analyzed using T-test, and p < 0.05 indicated that there was a statistical difference.

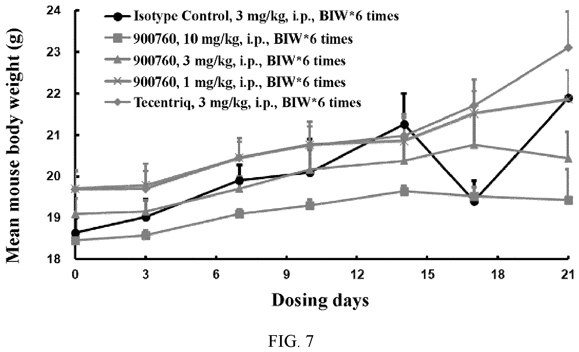

The results of changes in tumor volume and weight of mice after administration and treatment are shown in FIGs. 6 and 7 below. The humanized antibody 900760 has a dose-dependent anti-tumor effect which has been close to the maximum anti-tumor effect at a dose of 3 mg/kg, and a TGI of 76.89% which is close to the TGI of 78.15% in the 10 mg/kg dose group (see Table 3), which is better than the marketed reference drug Tecentriq with a TGI of 69.69%. The weight of the mice in each group continued to increase steadily, wherein the mice in the isotype negative control group were euthanized since Day 17 due to excessive tumor size, causing large fluctuation in the data of this group.

**Table 4. Analysis of Inhibitory Rate of Tumor Growth**

| Group | Tumor Volume on Day 14 (mm³) | TGI (%) | *p*-value |
|---|---|---|---|
| Isotype control, 3 mg/kg, i.p., BIW * 6 times | 3025.17±361.09 | - | - |
| 900760, 10 mg/kg, i.p., BIW * 6 times | 736.79±289.58 | 78.15% | ** |
| 900760, 3 mg/kg, i.p., BIW * 6 times | 773.24±342.16 | 76.89% | ** |
| 900760, 1 mg/kg, i.p., BIW * 6 times | 1233.65±165.01 | 61.18% | ** |
| Tecentriq, 3 mg/kg, i.p., BIW * 6 times | 984.36±130.18 | 69.69% | ** |

In Table 4, the data are expressed as Mean ± SEM; one-way ANOVA was used to compare the tumor volumes between the control group and the treatment group, ** means p < 0.01, * means p < 0.05, and ns means p > 0.05.

The foregoing detailed description is provided by way of explanation and examples and is not intended to limit the scope of the appended claims. Various changes of the embodiments currently set forth in this application are obvious to those of ordinary skills in the art and are reserved within the scope of the appended claims and their equivalents.

## Claims

1. An isolated antigen binding protein, having one or more of the following properties:
(1) capable of binding to a PD-L1 protein at an EC₅₀ value of 0.07 µg/ml or above;
(2) capable of blocking the binding of the PD-L1 protein to a PD-1 protein;
(3) capable of promoting the activation of a T cell; and
(4) capable of inhibiting the volume increase of a tumor in the body.

2. The isolated antigen binding protein of claim 1, comprising an antibody or an antigen binding fragment thereof.

3. The isolated antigen binding protein of claim 2, wherein the antigen binding fragment comprises Fab, Fab', F(ab)2, an Fv fragment, F(ab')2, scFv, di-scFv, VHH and/or dAb.

4. The isolated antigen binding protein of any one of claims 2-3, wherein the antibody is selected from the group consisting of a monoclonal antibody, a chimeric antibody, a humanized antibody, and a full human antibody.

5. The isolated antigen binding protein of any one of claims 1-4, competing with a reference antibody for binding to the PD-L1 protein, wherein the reference antibody comprises a heavy chain variable region VH and a light chain variable region VL, the VH of the reference antibody comprises a HCDR1, a HCDR2 and a HCDR3, the VL of the reference antibody comprises a LCDR1, a LCDR2 and a LCDR3, and the HCDR1 of the reference antibody comprises an amino acid sequence as set forth in SEQ ID NO: 1, the HCDR2 of the reference antibody comprises an amino acid sequence as set forth in SEQ ID NO: 21, the HCDR3 of the reference antibody comprises an amino acid sequence as set forth in SEQ ID NO: 22, the LCDR1 of the reference antibody comprises an amino acid sequence as set forth in SEQ ID NO: 6, the LCDR2 of the reference antibody comprises an amino acid sequence as set forth in SEQ ID NO: 23, and the LCDR3 of the reference antibody comprises an amino acid sequence as set forth in SEQ ID NO: 8.

6. The isolated antigen binding protein of any one of claims 1-5, comprising a HCDR1, and the HCDR1 comprises an amino acid sequence as set forth in SEQ ID NO: 1.

7. The isolated antigen binding protein of any one of claims 1-6, comprising a HCDR2, and the HCDR2 comprises an amino acid sequence as set forth in SEQ ID NO: 21.

8. The isolated antigen binding protein of any one of claims 1-7, comprising a HCDR2, and the HCDR2 comprises an amino acid sequence as set forth in SEQ ID NO: 2 or 4.

9. The isolated antigen binding protein of any one of claims 1-8, comprising a HCDR3, and the HCDR3 comprises an amino acid sequence as set forth in SEQ ID NO: 22.

10. The isolated antigen binding protein of any one of claims 1-9, comprising a HCDR3, and the HCDR3 comprises an amino acid sequence as set forth in SEQ ID NO: 3 or 5.

11. The isolated antigen binding protein of any one of claims 1-10, comprising a HCDR1, a HCDR2 and a HCDR3, wherein the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 3; or
wherein the HCDR1 comprises an amino acid sequence as set forth in SEQ ID NO: 1, the HCDR2 comprises an amino acid sequence as set forth in SEQ ID NO: 4, and the HCDR3 comprises an amino acid sequence as set forth in SEQ ID NO: 3; or
wherein the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 5; or
wherein the HCDR1 comprises an amino acid sequence as set forth in SEQ ID NO: 1, the HCDR2 comprises an amino acid sequence as set forth in SEQ ID NO: 4, and the HCDR3 comprises an amino acid sequence as set forth in SEQ ID NO: 5.

12. The isolated antigen binding protein of any one of claims 1-11, comprising a heavy chain variable region VH, wherein the VH comprises an amino acid sequence as set forth in any one of SEQ ID NOs: 11-16.

13. The isolated antigen binding protein of any one of claims 1-12, comprising a heavy chain constant region, and the heavy chain constant region is derived from a human IgG constant region.

14. The isolated antigen binding protein of claim 13, wherein the heavy chain constant region is derived from a human IgG1 constant region, and the human IgG1 constant region comprises an amino acid sequence as set forth in SEQ ID NO: 55.

15. The isolated antigen binding protein of any one of claims 1-14, comprising a LCDR1, and the LCDR1 comprises an amino acid sequence as set forth in SEQ ID NO: 6.

16. The isolated antigen binding protein of any one of claims 1-15, comprising a LCDR2, and the LCDR2 comprises an amino acid sequence as set forth in SEQ ID NO: 23.

17. The isolated antigen binding protein of any one of claims 1-16, comprising a LCDR2, and the LCDR2 comprises an amino acid sequence as set forth in SEQ ID NO: 7, 9 or 10.

18. The isolated antigen binding protein of any one of claims 1-17, comprising a LCDR3, and the LCDR3 comprises an amino acid sequence as set forth in SEQ ID NO: 8.

19. The isolated antigen binding protein of any one of claims 1-18, comprising a LCDR1, a LCDR2 and a LCDR3, wherein the LCDR1 comprises an amino acid sequence as set forth in SEQ ID NO: 6, the LCDR2 comprises an amino acid sequence as set forth in SEQ ID NO: 7, and the LCDR3 comprises an amino acid sequence as set forth in SEQ ID NO: 8; or
wherein the LCDR1 comprises an amino acid sequence as set forth in SEQ ID NO: 6, the LCDR2 comprises an amino acid sequence as set forth in SEQ ID NO: 9, and the LCDR3 comprises an amino acid sequence as set forth in SEQ ID NO: 8; or
wherein the LCDR1 comprises an amino acid sequence as set forth in SEQ ID NO: 6, the LCDR2 comprises an amino acid sequence as set forth in SEQ ID NO: 10, and the LCDR3 comprises an amino acid sequence as set forth in SEQ ID NO: 8.

20. The isolated antigen binding protein of any one of claims 1-19, comprising a light chain variable region VL, wherein the VL comprises an amino acid sequence as set forth in any one of SEQ ID NOs: 17-20.

21. The isolated antigen binding protein of any one of claims 1-20, comprising an antibody light chain constant region, and the antibody light chain constant region comprises an amino acid sequence as set forth in SEQ ID NO: 56.

22. The isolated antigen binding protein of any one of claims 1-21, comprising a heavy chain variable region VH and a light chain variable region VL, wherein,
1) the VH comprises an amino acid sequence as set forth in SEQ ID NO: 11, and the VL comprises an amino acid sequence as set forth in SEQ ID NO: 17, or
2) the VH comprises an amino acid sequence as set forth in SEQ ID NO: 12, and the VL comprises an amino acid sequence as set forth in SEQ ID NO: 18, or
3) the VH comprises an amino acid sequence as set forth in SEQ ID NO: 13, and the VL comprises an amino acid sequence as set forth in SEQ ID NO: 18, or
4) the VH comprises an amino acid sequence as set forth in SEQ ID NO: 14, and the VL comprises an amino acid sequence as set forth in SEQ ID NO: 18, or
5) the VH comprises an amino acid sequence as set forth in SEQ ID NO: 15, and the VL comprises an amino acid sequence as set forth in SEQ ID NO: 18, or
6) the VH comprises an amino acid sequence as set forth in SEQ ID NO: 16, and the VL comprises an amino acid sequence as set forth in SEQ ID NO: 18, or
7) the VH comprises an amino acid sequence as set forth in SEQ ID NO: 13, and the VL comprises an amino acid sequence as set forth in SEQ ID NO: 19, or
8) the VH comprises an amino acid sequence as set forth in SEQ ID NO: 14, and the VL comprises an amino acid sequence as set forth in SEQ ID NO: 19, or
9) the VH comprises an amino acid sequence as set forth in SEQ ID NO: 15, and the VL comprises an amino acid sequence as set forth in SEQ ID NO: 19, or
10) the VH comprises an amino acid sequence as set forth in SEQ ID NO: 16, and the VL comprises an amino acid sequence as set forth in SEQ ID NO: 19, or
11) the VH comprises an amino acid sequence as set forth in SEQ ID NO: 14, and the VL comprises an amino acid sequence as set forth in SEQ ID NO: 20, or
12) the VH comprises an amino acid sequence as set forth in SEQ ID NO: 15, and the VL comprises an amino acid sequence as set forth in SEQ ID NO: 20, or
13) the VH comprises an amino acid sequence as set forth in SEQ ID NO: 16, and the VL comprises an amino acid sequence as set forth in SEQ ID NO: 20.

23. A polypeptide, comprising the isolated antigen binding protein of any one of claims 1-22.

24. An immunoconjugate, comprising the isolated antigen binding protein of any one of claims 1-22 or the polypeptide of claim 23.

25. One or more isolated nucleic acid molecules, encoding the isolated antigen binding protein of any one of claims 1-22.

26. A vector, comprising the nucleic acid molecules of claim 25.

27. A cell, comprising the nucleic acid molecules of claim 25 or the vector of claim 26.

28. A method for preparing the isolated antigen binding protein of any one of claims 1-22, comprising culturing the cell of claim 27 under a condition enabling the expression of the isolated antigen binding protein of any one of claims 1-22.

29. A pharmaceutical composition, comprising the isolated antigen binding protein of any one of claims 1-22, the nucleic acid molecules of claim 25, the vector of claim 26 and/or the cell of claim 27, and optionally a pharmaceutically acceptable carrier.

30. Use of the isolated antigen binding protein of any one of claims 1-22, the polypeptide of claim 23, the immunoconjugate of claim 24, the nucleic acid molecules of claim 25, the vector of claim 26, the cell of claim 27 and/or the pharmaceutical composition of claim 29, the drug is used for preventing, alleviating, and/or treating a tumor.

31. The use of claim 30, wherein the tumor comprises a tumor with high expression of PD-1 or PD-L1.

32. The use of any one of claims 30-31, wherein the tumor comprises a solid tumor.

33. The use of any one of claims 30-32, wherein the tumor comprises colon cancer.

34. A method for preventing, alleviating, or treating a tumor, comprising administering to a subject in need thereof the isolated antigen binding protein of any one of claims 1-22, the polypeptide of claim 23, the immunoconjugate of claim 24, the nucleic acid molecules of claim 25, the vector of claim 26, the cell of claim 27 and/or the pharmaceutical composition of claim 29.

35. The method of claim 34, wherein the tumor comprises a tumor with high expression of PD-1 or PD-L1.

36. The method of any one of claims 34-35, wherein the tumor comprises a solid tumor.

37. The method of any one of claims 34-36, wherein the tumor comprises colon cancer.

38. The isolated antigen binding protein of any one of claims 1-22, the polypeptide of claim 23, the immunoconjugate of claim 24, the nucleic acid molecules of claim 25, the vector of claim 26, the cell of claim 27 and/or the pharmaceutical composition of claim 29, for use in preventing, alleviating, or treating a tumor.

39. A method for inhibiting the binding of PD-1 protein to PD-L1 protein, comprising administering the isolated antigen binding protein of any one of claims 1-22, the polypeptide of claim 23, the immunoconjugate of claim 24, the nucleic acid molecules of claim 25, the vector of claim 26, the cell of claim 27 and/or the pharmaceutical composition of claim 29.

40. A method for promoting the activation of a T cell, comprising administering the isolated antigen binding protein of any one of claims 1-22, the polypeptide of claim 23, the immunoconjugate of claim 24, the nucleic acid molecules of claim 25, the vector of claim 26, the cell of claim 27 and/or the pharmaceutical composition of claim 29.

41. A method for detecting the presence and/or content of PD-L1 protein, comprising administering the isolated antigen binding protein of any one of claims 1-22 and/or the polypeptide of claim 23.

42. A kit, comprising the isolated antigen binding protein of any one of claims 1-22, the polypeptide of claim 23, the immunoconjugate of claim 24, the nucleic acid molecules of claim 25, the vector of claim 26, the cell of claim 27 and/or the pharmaceutical composition of claim 29.
